(19) Europäisches Patentamt
European Patent Office
Office européen des brevets

(11) EP 4 371 589 A1

(12) EUROPEAN PATENT APPLICATION
published in accordance with Art. 153(4) EPC

(43) Date of publication:
**22.05.2024 Bulletin 2024/21**

(21) Application number: **22842073.3**

(22) Date of filing: **11.07.2022**

(51) International Patent Classification (IPC):
***A61M 1/18*** *(2006.01)* ***A61K 31/765*** *(2006.01)*
***A61M 1/36*** *(2006.01)* ***A61P 7/08*** *(2006.01)*
***B01D 71/52*** *(2006.01)* ***B01D 71/62*** *(2006.01)*
***B01D 71/68*** *(2006.01)*

(52) Cooperative Patent Classification (CPC):
**A61K 31/765; A61M 1/36; A61P 7/08; B01D 63/02; B01D 71/52; B01D 71/62; B01D 71/68**

(86) International application number:
**PCT/JP2022/027231**

(87) International publication number:
**WO 2023/286729 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2021 JP 2021116983**

(71) Applicants:
- **TOYOBO CO., LTD.**
  **Osaka-shi, Osaka 530-0001 (JP)**
- **KANEKA CORPORATION**
  **Osaka 530-8288 (JP)**

(72) Inventors:
- **KOYAMA, Shinya**
  **Otsu-shi, Shiga 520-0292 (JP)**
- **FUJITA, Koji**
  **Settsu-shi, Osaka 566-0072 (JP)**

(74) Representative: **Vossius & Partner**
**Patentanwälte Rechtsanwälte mbB**
**Siebertstrasse 3**
**81675 München (DE)**

# (54) SEMIPERMEABLE MEMBRANE

(57) A semipermeable membrane comprising a polyether sulfone, a sulfonated polyarylene ether copolymer, and polyvinylpyrrolidone, having cytokine adsorption capabilities suitable for continuous renal replacement therapy. The sulfonated polyarylene ether copolymer includes a hydrophobic segment repeating unit represented by a formula (1) and a hydrophilic segment repeating unit represented by a formula (2) as copolymerization components. A ratio of (the polyether sulfone)/(the sulfonated polyarylene ether copolymer)/(the polyvinylpyrrolidone) (in mass) in the entirety of the semipermeable membrane is (75 to 90)/(7 to 22)/(3 to 18).

FIG.1

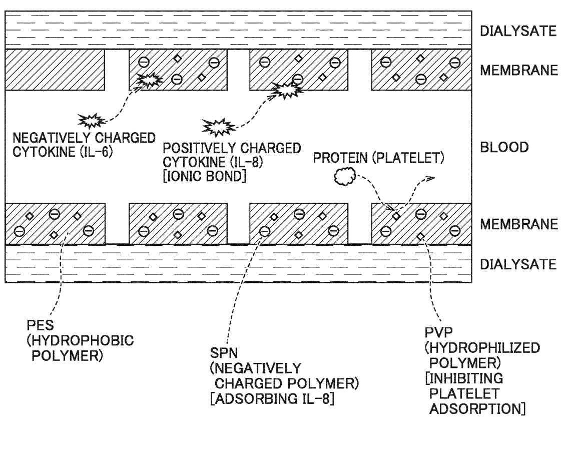


EP 4 371 589 A1

## Description

TECHNICAL FIELD

The present invention relates to a semipermeable membrane.

BACKGROUND ART

There is a therapy known as continuous renal replacement therapy (CRRT), which is for acute renal failure patients, sepsis patients, and the like and which involves performing body fluid correction over a prolonged period of time in a slow manner (at a lower circulation flow rate) as compared to the therapy conditions for ordinary hemodialysis and hemofiltration. For instance, a single-round treatment of CRRT is carried out at a blood flow rate from 50 to 150 mL/minute over 24 to 48 hours.

Sepsis occurs primarily at the time of infection with a pathogen such as bacteria and/or viruses when the pathogen penetrates into blood vessels to cause an increase of proinflammatory cytokines in blood, and it becomes serious when cytokine storm occurs (which is a systemic inflammation reaction caused by proinflammatory cytokines). Hence, in CRRT treatment of sepsis and/or the like, it is necessary to remove proinflammatory cytokines from blood.

In a continuous hemofiltration instrument used in CRRT, a semipermeable membrane with cytokine adsorption capabilities is used. As the continuous hemofiltration instrument, SepXiris (registered trademark) manufactured by Baxter Limited is known, for example. The semipermeable membrane used in SepXiris contains, as a component, a copolymer of acrylonitrile and sodium methallyl sulfonate, as shown in Fig. 8. It is conceivable that this semipermeable membrane is mainly capable of adsorbing positively charged cytokines because its negatively charged sulfonate groups can form ionic bonds with amino groups of positively charged cytokines (see NPL 1 (The Journal of Artificial Organs, vol. 43, No. 3, pp. 233-237, 2014)).

Moreover, PTL 1 (Japanese Patent Laying-Open No. 2011-62282) discloses a hollow fiber membrane for hemodiafiltration purposes that has hemocompatibility and cytokine adsorption capabilities. This hollow fiber membrane contains polymethyl methacrylate and sodium p-styrenesulfonate, and due to the negatively charged sulfonate groups, it is conceivable that the membrane is mainly capable of adsorbing positively charged cytokines.

Furthermore, PTL 2 (Japanese Patent Laying-Open No. 2001-70767) discloses an ultrafiltration membrane for surface water treatment purposes, consisting of a composition containing polyether sulfone, polyvinylpyrrolidone, and sulfonated polyether sulfone.

CITATION LIST

PATENT LITERATURE

PTL 1: Japanese Patent Laying-Open No. 2011-62282
PTL 2: Japanese Patent Laying-Open No. 2001-70767

NON PATENT LITERATURE

NPL 1: The Journal of Artificial Organs, vol. 43, No. 3, pp. 233-237, 2014

SUMMARY OF INVENTION

TECHNICAL PROBLEM

The above-mentioned conventional semipermeable membranes for cytokine removal purposes are highly capable of adsorbing positively charged cytokines such as interleukin-8 (IL-8), but also have a drawback of low capabilities in adsorbing negatively charged cytokines such as interleukin-6 (IL-6).

Hence, an object of the present invention is to provide a semipermeable membrane for cytokine removal purposes that is highly capable of adsorbing both negatively charged cytokines and positively charged cytokines.

SOLUTION TO PROBLEM

[1] A semipermeable membrane comprising:

a polyether sulfone;
a sulfonated polyarylene ether copolymer; and
polyvinylpyrrolidone, wherein
the sulfonated polyarylene ether copolymer includes a hydrophobic segment repeating unit represented by the following formula (1) and a hydrophilic segment repeating unit represented by the following formula (2) as copolymerization components,

[Chemical 1]

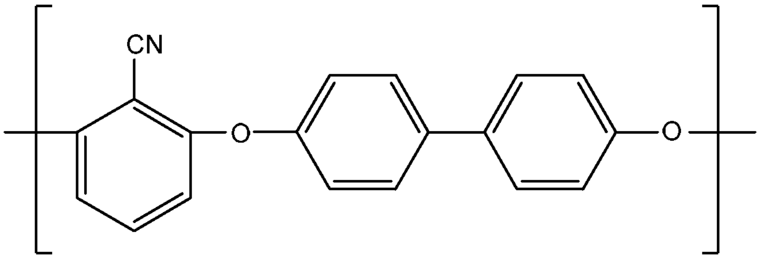

(1)

[Chemical 2]

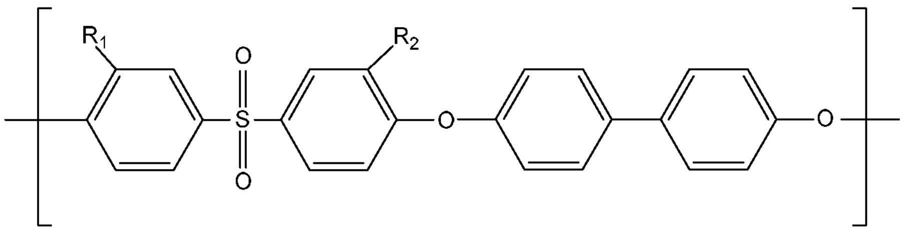

(2)

a component ratio (in mole) of the formula (1) is from 0.50 to 0.65, a component ratio (in mole) of the formula (2) is from 0.35 to 0.50, a sum of the component ratio (in mole) of the formula (1) and the component ratio (in mole) of the formula (2) is 1.00, each of $R_1$ and $R_2$ represents "$-SO_3M$", M represents a metallic element,
a ratio of (the polyether sulfone)/(the sulfonated polyarylene ether copolymer)/(the polyvinylpyrrolidone) (in mass) in the entirety of the semipermeable membrane is (75 to 90)/(7 to 22)/(3 to 18), and
the semipermeable membrane has cytokine adsorption capabilities suitable for continuous renal replacement therapy.

[2] The semipermeable membrane according to [1], wherein the semipermeable membrane has a structure that is not uniform in a thickness direction.
[3] The semipermeable membrane according to [1] or [2], wherein the semipermeable membrane has a dense layer on an inner surface side thereof.
[4] The semipermeable membrane according to any one of [1], [2], or [3], wherein a NaOH titration amount for the entirety of the semipermeable membrane is from 1.2 to 3.0 mL.

ADVANTAGEOUS EFFECTS OF INVENTION

The present invention makes it possible to provide a semipermeable membrane for cytokine removal purposes that is highly capable of adsorbing both negatively charged cytokines and positively charged cytokines.

BRIEF DESCRIPTION OF DRAWINGS

Fig. 1 is a schematic view illustrating a semipermeable membrane for cytokine removal purposes according to an embodiment.
Fig. 2 is a graph showing the relationship between the SPN content and the IL-8 adsorption rate.

Fig. 3 is a graph showing the relationship between the SPN content and the IL-6 adsorption rate.
Fig. 4 is a graph showing the relationship between the PVP content and the platelet remaining rate.
Fig. 5 is a graph showing the relationship between the NaOH titration amount and the IL-8 adsorption rate.
Fig. 6 is a graph showing results of filtration stability evaluation.
Fig. 7 is a graph showing the relationship between the PVP content and the CLmyo MBE.
Fig. 8 is a schematic view illustrating a conventional semipermeable membrane for cytokine removal purposes.
Fig. 9 shows SEM photographs of a hollow fiber membrane according to an embodiment.

DESCRIPTION OF EMBODIMENTS

In the following, a description will be given of embodiments of the present invention, but the scope of the present invention is not limited to these embodiments.

<Semipermeable Membrane for Cytokine Removal Purposes>

A semipermeable membrane according to the present embodiment is a semipermeable membrane for cytokine removal purposes that comprises a polyether sulfone (a hydrophobic polymer), a sulfonated polyarylene ether copolymer (a negatively charged polymer), and polyvinylpyrrolidone (a hydrophilic polymer).

Examples of the cytokines include positively charged cytokines and negatively charged cytokines. Examples of the positively charged cytokines include interleukin-8 (IL-8), interleukin-10 (IL-10), transforming growth factor β (TGF-β), monocyte chemotaxis promoting factor (MCP1), and platelet-derived growth factor (PDGF). Examples of the negatively charged cytokines include interleukin-6 (IL-6), interleukin-18 (IL-18), interleukin-1β (IL-1β), HMGB1 (High Mobility Group Box 1), and tumor necrosis factor-α (TNF-α). Examples of the cytokines include proinflammatory cytokines which can cause various inflammatory symptoms in living things.

Referring to Fig. 1, it is conceivable that a positively charged cytokine such as IL-8 can form an ionic bond with a sulfonated polyarylene ether copolymer (SPN) which is a negatively charged polymer, and then be adsorbed on the semipermeable membrane.

It is conceivable that polyvinylpyrrolidone which is a hydrophilicity-rendering agent (a hydrophilic polymer) can inhibit adsorption of proteins such as platelets onto the semipermeable membrane.

Although the action mechanism is unclear, the semipermeable membrane according to the present embodiment has enhanced capabilities of adsorbing negatively charged cytokines such as IL-6 as compared to conventional semipermeable membranes.

(Polyether Sulfone)

The polyether sulfone is a compound that includes a structural unit represented by the following formula (3). The polyether sulfone is expected to act to adsorb proinflammatory cytokines via hydrophobic interaction.

[Chemical 3]

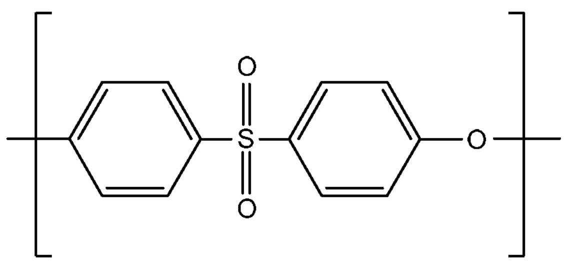

(3)

The sulfonated polyarylene ether copolymer is a compound that includes a hydrophobic segment repeating unit represented by the following formula (1) and a hydrophilic segment repeating unit represented by the following formula (2).

[Chemical 4]

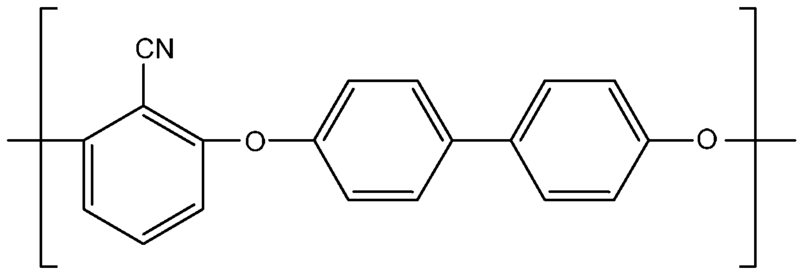

(1)

[Chemical 5]

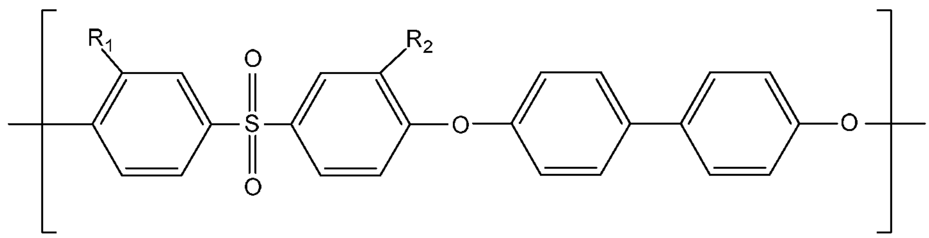

(2)

The component ratio (in mole) of the formula (1) is from 0.50 to 0.65; the component ratio (in mole) of the formula (2) is from 0.35 to 0.50; the sum of the component ratio (in mole) of the formula (1) and the component ratio (in mole) of the formula (2) is 1.00; $R_1$ and $R_2$ independently represent "$-SO_3M$" or "$-SO_3H$"; and M represents a metallic element.

The sulfonated polyarylene ether copolymer may be any of a random copolymer, a block copolymer, an alternating copolymer, and the like.

M (a metallic element) is not particularly limited, and examples thereof include sodium, potassium, and lithium.

In the sulfonated polyarylene ether copolymer, it is more preferable that the component ratio (in mole) of the formula (1) be from 0.50 to 0.60 and the component ratio (in mole) of the formula (2) be from 0.40 to 0.50, and it is more preferable that each of $R_1$ and $R_2$ in the formula (2) be "$-SO_3Na$" or "$-SO_3K$".

The sulfonated polyarylene ether copolymer is a material that does not change much during a long-term use of the membrane.

The sulfonated polyarylene ether copolymer may be obtained by a conventionally known method and the like. For instance, 2,6-dichlorobenzonitrile, 3,3'-disulfo-4,4'-dichlorodiphenyl sulfone metal salt, and 4,4'-biphenol may be subjected to reaction in the presence of a basic compound for polymerization via an aromatic nucleophilic substitution reaction, and thereby a copolymer consisting of a hydrophobic segment represented by the formula (1) and a hydrophilic segment represented by the formula (2) may be obtained.

The polymerization may be caused at a temperature within the range of 0 to 350°C, and, preferably, the temperature is from 50 to 250°C. At below 0°C, the reaction tends not to proceed sufficiently, and at above 350°C, polymer degradation tends to begin.

The reaction may be caused in the absence of solvent, but preferably caused in solvent. Examples of the solvent that can be used include N-methyl-2-pyrrolidone, N,N-dimethylacetamide, N,N-dimethylformamide, dimethyl sulfoxide, diphenyl sulfone, and sulfolane; however, these examples are not restrictive and any solvent that is stable in an aromatic nucleophilic substitution reaction may be used.

Examples of the basic compound include sodium hydroxide, potassium hydroxide, sodium carbonate, potassium carbonate, sodium hydrogen carbonate, potassium hydrogen carbonate, and the like; however, these examples are not restrictive and those that can transform an aromatic diol into an active phenoxide structure may be used.

In an aromatic nucleophilic substitution reaction, water can be produced as a byproduct. In that case, toluene and/or the like may be made to coexist in the reaction system in addition to and independently from the polymerization solvent to remove water as an azeotrope, out of the system. As the method for removing water out of the system, use of a water-absorbing material such as a molecular sieve may also be adopted.

In the case when the aromatic nucleophilic substitution reaction is caused in a solvent, it is preferable that the monomers be added in such a manner that a resulting polymer concentration from 5 to 50 mass% is to be achieved. When it is below 5 mass%, the degree of polymerization tends not to increase. When it is above 50 mass%, the viscosity of the reaction system tends to become too high to perform posttreatment of the reaction product with ease.

After the completion of the polymerization reaction, the solvent is evaporated and removed from the reaction

solution, followed by rinsing of the remaining product as needed, to obtain a desired polymer (a sulfonated polyarylene ether copolymer). Alternatively, the reaction solution may be added to a solvent that does not dissolve the polymer very well, to make the polymer precipitated in solid form and collect the precipitate by filtration to obtain the polymer.

(Polyvinylpyrrolidone)

The polyvinylpyrrolidone is a polymer of monomers including at least N-vinylpyrrolidone.
The polyvinylpyrrolidone preferably includes a structural unit represented by the following formula (4).

[Chemical 6]

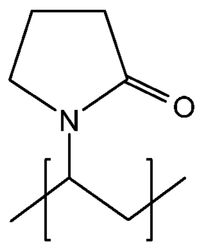

(4)

As the polyvinylpyrrolidone, products from BASF with weight average molecular weights of 9,000 (K17), 450,000 (K60), and/or 1,200,000 (K90) can be used, for example.
Preferably, the ratio of (polyether sulfone)/(sulfonated polyarylene ether copolymer)/(polyvinylpyrrolidone) in the entirety of the semipermeable membrane is (75 to 90)/(7 to 22)/(3 to 18) (in mass%).
Preferably, the NaOH titration amount for the entirety of the semipermeable membrane is from 1.2 to 3.0 mL.
Preferably, the semipermeable membrane has a structure that is not uniform in a thickness direction (an asymmetric structure). More preferably, it has a structure that has a dense layer on the inner surface side thereof, with the pore size increasing continuously or discontinuously from the inner surface toward the outer surface (an asymmetric structure).
Examples of the semipermeable membrane having an asymmetric structure (an asymmetric membrane) include a membrane in which the above-mentioned dense layer serves as a separation-activating layer that substantially determines the pore size of the semipermeable membrane. It is impossible to directly measure the pore size of the dense layer, and the molecular weight cut-off of SC dextran measured with the use of a semipermeable membrane having a dense layer is defined as the pore size of the dense layer. In the present invention, the molecular weight cut-off of SC dextran measured in this manner is preferably 100 kDa or less. From the viewpoint of durability of the membrane (filtration stability), the semipermeable membrane is preferably an asymmetric membrane.
In a hollow fiber membrane having an asymmetric structure, the aperture ratio of the inner surface is preferably different from the aperture ratio of the outer surface. The aperture ratio of the outer surface of the hollow fiber membrane is preferably 29% or more, more preferably 30% or more. The aperture ratio of the outer surface of the hollow fiber membrane is determined in an image that is taken with a scanning electron microscope (SEM), as described below.
Examples of the semipermeable membrane include semipermeable membranes called nanofiltration membranes (NF membranes), ultrafiltration membranes (UF membranes), and microfiltration membranes (MF membranes). The semipermeable membrane is preferably an ultrafiltration membrane and/or a microfiltration membrane.
Usually, the pore size of an NF membrane is from about 1 to 2 nm, the pore size of an UF membrane is from about 2 to 100 nm, and the pore size of an MF membrane is 0.1 μm or more.
The shape of the semipermeable membrane is not particularly limited. The semipermeable membrane may be a flat membrane or a hollow fiber membrane (a hollow-fiber-type semipermeable membrane), and is preferably a hollow fiber membrane. A hollow fiber membrane has a small membrane thickness and provides an increased membrane area per module, as compared to a flat membrane, thereby having an enhanced cytokine removal efficiency, which is an advantage.
In a semipermeable membrane module (a continuous hemofiltration instrument) having the above-mentioned hollow fiber membrane, it is preferable that the dialysate be made to flow on the outside of the hollow fiber membrane and blood be made to flow on the inner side (inside the hollow portion) of the hollow fiber membrane (see Fig. 1).

[Examples]

In the following, a more detailed description will be given of the present invention by way of Examples, but these Examples are not intended to limit the scope of the present invention.

(Preparation of Sulfonated Polyarylene Ether Copolymer)

3,3'-disulfo-4,4'-dichlorodiphenyl sulfone disodium salt, 2,6-dichlorobenzonitrile, 4,4'-biphenol, potassium carbonate, and a molecular sieve were weighed into a four-necked flask, through which nitrogen was passed. NMP was added thereto and the resultant was stirred at 150°C for 50 minutes, followed by increasing the reaction temperature to the range of 195°C to 200°C to continue reaction until the viscosity of the system rose sufficiently. The resultant was left to cool, and after cooling, the sedimented molecular sieve was removed and the polymer was precipitated in water. The polymer thus obtained was rinsed in boiling water for 1 hour, followed by careful rinsing in deionized water to completely remove the remaining potassium carbonate. Subsequently, the polymer from which potassium carbonate had been removed was dried, and thereby a sulfonated polyarylene ether copolymer was obtained.

(Preparation of Hollow Fiber Membrane of Example 1)

16.0 mass% of polyether sulfone (PES, manufactured by Sumitomo Chemical), 4.0 mass% of sulfonated polyarylene ether copolymer (SPN, manufactured by Toyobo), 3.0 mass% of polyvinylpyrrolidone K-90 (PVP, manufactured by Nippon Shokubai), 46.2 mass% of N-methyl-2-pyrrolidone (NMP, manufactured by Mitsubishi Chemical), and 30.8 mass% of triethylene glycol (TEG, manufactured by Mitsubishi Chemical) were heated and dissolved into uniformity. The spinning dope thus obtained, together with a hollow-forming material (NMP/TEG/water=12/8/80), was discharged through a tube-in-orifice nozzle that was warmed to 55°C, passed through a 200-mm dry section that was shut off from the outside air by a spinning tube, coagulated in a coagulation bath (NMP/TEG/water=24/16/60) at 74°C, rinsed in a water bath at 80°C, and wound on a skeiner at a spinning rate of 37 m/min.

The hollow fiber membrane thus obtained had a dense layer on the inner surface side thereof, an inner diameter of 0.240 mm, and an outer diameter of 0.350 mm.

The SPN used here is a copolymer of the formula (1) and the formula (2), with the component molar ratio of (1) being 0.56 and the component molar ratio of (2) being 0.44.

(Preparation of Hollow Fiber Membrane of Example 2)

A hollow fiber membrane having a dense layer on the inner surface side thereof was obtained under the same conditions as those employed for Example 1 except that some contents were changed as follows: 2.0 mass% of PVPK-90, 46.8 mass% of NMP, and 31.2 mass% of TEG.

(Preparation of Hollow Fiber Membrane of Example 3)

A hollow fiber membrane having a dense layer on the inner surface side thereof was obtained in the same manner as in Example 1 except that some contents were changed as follows: 16.0 mass% of PES (Sumitomo Chemical), 3.0 mass% of SPN (manufactured by Toyobo), 1.0 mass% of PVPK-90 (manufactured by Nippon Shokubai), 48.0 mass% of NMP (manufactured by Mitsubishi Chemical), and 32.0 mass% of TEG (manufactured by Mitsui Chemicals).

(Preparation of Hollow Fiber Membrane of Example 4)

A hollow fiber membrane having a dense layer on the inner surface side thereof was obtained in the same manner as in Example 1 except that some contents were changed as follows: 16.0 mass% of PES (Sumitomo Chemical), 2.0 mass% of SPN (manufactured by Toyobo), 2.0 mass% of PVPK-90 (manufactured by Nippon Shokubai), 48.0 mass% of NMP (manufactured by Mitsubishi Chemical), and 32.0 mass% of TEG (manufactured by Mitsui Chemicals).

(Preparation of Hollow Fiber Membrane of Comparative Example 2)

16.0 mass% of PES (Sumitomo Chemical), 4.0 mass% of SPN, 48.0 mass% of NMP, and 32.0 mass% of TEG were heated and dissolved into uniformity. The spinning dope thus obtained was used under the same conditions as in Example 1 to produce a hollow fiber membrane.

The hollow fiber membrane thus obtained had an inner diameter of 0.240 mm and an outer diameter of 0.350 mm.

(Preparation of Hollow Fiber Membrane of Comparative Example 3)

A spinning dope was prepared by kneading and dissolving 17.0 mass% of PES (manufactured by BASF, 6020P), 3.0 mass% of PVPK90 (manufactured by BASF), 75.0 mass% of dimethylacetamide as a solvent, and 5.0 mass% of RO water as a nonsolvent, at 60°C. The spinning dope thus obtained, together with a bore liquid (50 mass%

dimethylacetamide aqueous solution), was discharged through a double-tube nozzle that was warmed to 60°C, passed through a 400-mm dry section that was shut off from the outside air by a spinning tube, coagulated in 30 mass% dimethylacetamide aqueous solution at 70°C, passed through a water bath at 75°C, and wound on a reel at a rate of 30 m/min.

(Preparation of Hollow Fiber Membrane of Comparative Example 4)

21.0 mass% of PES (manufactured by Sumitomo Chemical), 35.55 mass% of NMP (manufactured by Mitsubishi Chemical), and 43.45 mass% of TEG (manufactured by Mitsui Chemicals) were heated and dissolved into uniformity. The spinning dope thus obtained, together with a hollow-forming material (NMP/TEG/water=36/24/40), was discharged through a tube-in-orifice nozzle that was warmed to 70°C, passed through a 40-mm dry section that was shut off from the outside air by a spinning tube, coagulated in a coagulation bath (NMP/TEG/water=3.6/2.4/94) at 75°C, rinsed in a water bath at 80°C, and wound on a skeiner at a spinning rate of 44 m/min.

The hollow fiber membrane thus obtained had an inner diameter of 0.210 mm and an outer diameter of 0.340 mm.

(Preparation of Semipermeable Membrane Module)

Both ends of the hollow fiber membrane of each Example, in the form of a bundle of 8080 hollow fiber membranes, were bonded to a module case to prepare a semipermeable membrane module. The resulting semipermeable membrane module (a continuous hemofiltration instrument) was used in the evaluation tests described below.

(Comparative Example 1)

As a semipermeable membrane module of Comparative Example 1, a continuous hemofiltration instrument manufactured by Baxter Limited [SepXiris (registered trademark), SepXiris 150] was prepared. The main component material of the hollow fiber membrane used in this continuous hemofiltration instrument is a copolymer of acrylonitrile and sodium methallyl sulfonate (Fig. 8).

<Evaluation Tests>

(Measurement of Aperture Ratio of Outer Surface)

An image of the surface of the hollow fiber membrane is taken at a magnification of 10,000 times. This image is processed with image analysis software to determine the aperture ratio of the surface of the hollow fiber membrane.

The area ratio is calculated by a method using image analysis. More specifically, image analysis software WinROOF2013 is used for the measurement. Pores are distinguished from polymer portions based on brightness, and undistinguished portions and noises are corrected with a freehand tool. Edge portions which are the outline of the pores, as well as porous structures observed in the back of the pores, are recognized as pores. The area of the measurement region (the region under measurement) (A) and the cumulative area of pores within the measurement region (B) are determined to calculate the aperture ratio of the outer surface (%)=B/A×100. The aperture ratio of the outer surface of the hollow fiber membrane obtained in Example 1 and SEM photographs (of the outer surface, the inner surface, and a cross section thereof) are shown in Table 2 and Fig. 9, respectively.

(Method for Measuring Hollow Fiber Membrane by $^1$H-NMR)

Measurement was carried out with the apparatus described below under the conditions described below.

(Apparatus): Fourier transformation nuclear magnetic resonance spectrometer (AVANCENEO 600 manufactured by Bruker Biospin)
(Measurement solution): 5 to 50 mg of sample dissolved in 0.6 mL of deuterated dimethyl sulfoxide.
(1H resonance frequency): 600.13 MHz
(Flip angle of detection pulse): 30°
(Data capture duration): 4.0 seconds
(Delay time): 1.0 seconds
(Number of transients): 5 to 200 times
(Measurement temperature): room temperature or 40°C

As for the hollow fiber membranes of Examples 1, 2 and Comparative Examples 2 to 5, with the use of NMR,

the composition ratios (the contents in the entirety of the hollow fiber membrane) of PES, SPN hydrophobic segment (a hydrophobic moiety), SPN hydrophilic segment (a hydrophilic moiety), and PVP were measured. Result of the measurement are shown in Table 1.

(Cytokine Adsorption Rate)

To 1000 mL of porcine whole blood to which heparin had been added, 24 μg of each of IL-6 and IL-8 was added to prepare a sample blood.
Through the semipermeable membrane module (a continuous hemofiltration instrument) of Examples 1, 2 and Comparative Examples 1 to 5, the sample blood was circulated for 1 hour under conditions of a blood flow rate (Qb) of 100 mL/minute and a filtrate flow rate (Qf) of 10 mL/minute.
After circulation, the entire amount of the sample blood was collected, and the amounts of IL-6 and IL-8 in the sample blood were measured by enzyme-linked immunosorbent assay (ELISA). From the measured values and the amounts added to the sample blood, decrements of IL-6 and IL-8 were calculated, which were regarded as the adsorption rates of IL-6 and IL-8. The IL-6 and IL-8 adsorption rates thus calculated are shown in Table 1.

(NaOH Titration Amount)

0.1 g of the hollow fibers obtained in Examples 1, 2 and Comparative Examples 2, 5 was weighted and rinsed with deionized water. Sulfone groups of sulfonated polyarylene ether copolymer of the hollow fibers thus rinsed were immersed in 5 mL of 1-M HCl aqueous solution for protonation. The hollow fibers were rinsed with deionized water, and then transferred to 80 mL of 1-M NaCl aqueous solution, followed by addition of 1 mL of 1% phenolphthalein solution thereto. Under stirring with the use of a stirrer, titration was carried out with 0.01-M NaOH aqueous solution to measure the NaOH titration amount (Reference: J. Chromatogr. A 1559 (2019) 152-160). Results of measurement of the NaOH titration amount are shown in Table 1.

(Platelet Remaining Rate)

As for Examples 1, 2 and Comparative Examples 1 to 5, 1000 mL of porcine whole blood to which heparin had been added was circulated for 1 hour under conditions of a blood flow rate (Qb) of 100 mL/minute and a filtrate flow rate (Qf) of 10 mL/minute. After circulation, the entire amount of the sample blood was collected. With the use of a blood cell counter, platelets in the blood after 1-hour circulation and those in the blood before circulation were measured. The resulting measured values were used for calculation of (platelet remaining rate)=(blood after 1-hour circulation)/(blood before circulation). Results of measurement of the platelet remaining rate are shown in Table 1.

[Table 1]

| | Supplied amount (parts by mass) | | | Composition ratio [NMR measured values] (mass%) | | | | Evaluation results | | | |
|---|---|---|---|---|---|---|---|---|---|---|---|
| | PES | SPN | PVP | PES | SPN | | PVP | NaOH titration amount (mL) | Cytokine adsorption rate (%) | | Platelet remaining (%) |
| | | | | | Hydrophobic moiety | Hydrophilic moiety | | | IL-6 | IL-8 | |
| Ex.1 | 16 | 4 | 3 | 79.6 | 4.2 | 7 | 9.2 | 2.9 | 91 | 94 | 84 |
| Ex.2 | 16 | 4 | 2 | 78.4 | 5.4 | 8.6 | 7.6 | 2.8 | 98 | 85 | 84 |
| Ex3 | 16 | 3 | 1 | 87.3 | 3.5 | 5.5 | 3.7 | 2.3 | 87 | 74 | 49 |
| Ex.4 | 16 | 2 | 2 | 88.3 | 2.1 | 3.5 | 6.0 | 1.4 | 82 | 54 | 76 |
| Comp.Ex.1 (conventional product) | - | - | - | - | - | - | - | - | 73 | 91 | 53 |
| Comp.Ex.2 | 16 | 4 | 0 | 87.9 | 4.8 | 7.3 | 0 | 2.9 | 92 | 95 | 25 |
| Comp.Ex.3 | 17 | 0 | 3 | - | - | - | - | - | 34 | 10 | 70 |
| Comp.Ex.4 | 16 | 0 | 0 | - | - | - | - | 0.0 | 87 | 34 | 10 |

Referring to Table 1, the adsorption rate of negatively charged cytokine interleukin 6 (IL-6) in Examples 1 to 4 was 74% or more, which was higher than that in Comparative Example 1 (a conventional product).

The adsorption rate of positively charged cytokine interleukin 8 (IL-8) was relatively high in Examples, 80% or more in Examples 1 and 2, higher in Example 1 than in Comparative Example 1 (a conventional product).

Fig. 2 shows the relationship between the SPN content and the IL-8 adsorption rate in Examples 1 to 4 and Comparative Examples 2 to 4. Referring to Fig. 2, the IL-8 adsorption rate increases as the SPN content increases. Here, from the graph of Fig. 2, it is conceivable that the SPN content is preferably from 7.0 to 22.0 mass% for achieving an IL-8 adsorption rate of 60% or more.

These results indicate that adsorption of positively charged cytokine IL-8 is related to the content of negatively charged SPN; however, from the relationship between Examples 1, 2 and Comparative Example 2 as well as the relationship between Comparative Example 3 and Comparative Example 4, it is suggested that PVP may somewhat affect IL-8 adsorption (that is, it is suggested that IL-8 adsorption may be inhibited by PVP addition).

Fig. 3 shows the relationship between the SPN content and the IL-6 adsorption rate in Examples 1 to 4 and Comparative Examples 2 to 4. Referring to Fig. 3, the IL-6 adsorption rate does not change much as the SPN content increases, and, as in the case of Comparative Example 4 shown in Table 1, IL-6 adsorbs on hollow fibers that are solely composed of polyether sulfone.

These results indicate that adsorption of negatively charged cytokine IL-6 is greatly affected by hydrophobic interaction with the SPN hydrophobic segment and with PES, although the specific action mechanism of IL-6 adsorption is unclear.

Fig. 4 shows the relationship between the PVP content and the platelet remaining rate in Examples 1 to 4 and Comparative Examples 2, 4. Referring to Fig. 4, the platelet remaining rate is 70% or more in Examples 1, 2, 4 where the PVP content is 6.0% or more, indicating that it is preferable that the PVP content be more than 3.0% for achieving a platelet remaining rate of 45% (an index value indicating the requirement in the market) or more.

For a semipermeable membrane for use in CRRT and the like, it is desirable that the adsorption of blood components such as platelets be low and the platelet remaining rate be high.

(Amount of Negative Charge)

The hollow fiber membranes of Examples 1 to 4 and Comparative Examples 2, 4 were subjected to measurement of NaOH titration amount as described below, as an index of the amount of negative charge.

(1) NaOH Titration Amount

Sulfone groups of the sulfonated polyarylene ether copolymer of the hollow fiber membrane were protonated with HCl, followed by rinsing and then NaOH titration, and thus the NaOH titration amount was measured (Reference: J. Chromatogr. A 1559(2019)152-160).

Fig. 5 shows the relationship between the NaOH titration amount and the IL-8 adsorption rate in Examples 1 to 4 and Comparative Examples 2, 4.

The results shown in Fig. 5 indicate that the adsorption rate of the positively charged cytokine (IL-8) increases as the amount of negative charge (the NaOH titration amount and the amount of sulfone groups) increases. Here, it is conceivable that referring to the graph in Fig. 5, the NaOH titration amount is preferably 1.2 mL or more for achieving an IL-8 adsorption rate of 50% or more.

(Filtration Stability: ΔTMP)

Through the hollow fiber membrane module of Example 1 and Comparative Example 1, bovine blood which contained citric acid added thereto for coagulation inhibition and which had been adjusted to a hematocrit content of 30±2% and a protein concentration of 6 to 7 g/dL was circulated for 150 minutes under conditions of a blood flow rate (Qb) of 400 mL/minute and a filtration flow rate (Qf) of 100 mL/minute. A value of TMP (the measured value after a lapse of a specific time period up to 150 minutes) from which TMP after a lapse of 15 minutes was subtracted was defined as ΔTMP. TMP (transmembrane pressure) is as described below. Results of the measurement are shown in Fig. 6. Results of the measurement of ΔTMP in Example 1 and Comparative Example 1 after a lapse of 150 minutes are shown in Table 2.

(Performance in Aqueous System)

As for Example 1 and Comparative Examples 1, 2, 5, the below-described performances (1) to (3) (performance in aqueous system) were measured. Results of the measurement are shown in Table 2.

(1) (Measurement of Ultrafiltration Coefficient UFR of Deionized Water)

The blood outlet circuit of the hollow fiber membrane module was sealed for dead-end filtration. A pressure tank was filled with deionized water maintained at 37°C and, while the pressure was regulated with a regulator, the deionized water was fed into the inside of the hollow fiber membrane of the hollow fiber membrane module placed inside a thermostatic chamber maintained at 37°C and the amount of filtrate flowing out to the outside of the hollow fiber membrane was measured with a measuring cylinder. The transmembrane pressure (TMP) was set at TMP=(Pi+Po)/2. Pi is the pressure at the module inlet, and Po is the pressure at the module outlet. TMP was changed and the filtration flow rate was measured at four positions; from the slope of the straight line thus obtained, the permeability UFR (mL/hr/$m^2$/mmHg) of the module was calculated. At this point, the correlation coefficient between TMP and the filtration flow rate needs to be 0.999 or more. For reducing the pressure loss error attributable to the circuit, measurement was carried out at a TMP within the range of 100 mmHg or less.

(2) (Measurement of Clearance (CL))

According to "The method of hemodialyzer performance assessment, 2012", clearance (CL) of urea (UN), vitamin B12 (Vb), and myoglobin (myo) was measured. Clearance (CL) and mass balance error (MBE) thereof are shown in Table 2. Clearance (CL) is an index of substance removal, and mass balance error (MBE) is an index of measurement errors.

Physiological saline in which 0.1% urea (manufactured by Nacalai Tesque), 0.002% vitamin B12 (manufactured by Nacalai Tesque), and 0.01% myoglobin (manufactured by Kishida Chemical) were dissolved was made to flow single-pass at a flow rate (QBin) of 200 mL/min, without being filtered, through the blood channel side of a hemodialyzer (membrane area, 1.5 m2) which had been primed and wetted with physiological saline, and through the dialysate-side channel, the dialysate was made to flow at a flow rate (Qd) of 500 mL/min. The solute concentration of the measurement liquid at the inlet of the hollow fiber membrane, the solute concentration of the measurement liquid at the outlet, and the amount of liquid supply were measured to calculate clearance (CL).

Clearance (CL) is represented by the following equation.

CL (mL/min)=(((inlet concentration)-(outlet concentration))/(inlet concentration))×(amount of liquid supply)

Mass balance error (MBE) is represented by the following equation.

$$MBE=(MB-MD)/MB$$

*MB=(blood-side inlet flow rate)×((inlet concentration)-(outlet concentration)),

MD=(dialysate flow rate)×(dialysate concentration)

(3) (Calculation of Protein Leak Amount (Total Protein Loss: TPL))

Bovine plasma with added citric acid was adjusted to a protein concentration of 6 to 7 g/dL and fed to the hollow fiber membrane module at 37°C at 200 mL/min for plasma filtration at a filtration flow rate of 15 mL/min. The filtrate was returned back to the plasma for circulation. The filtration flow rate was measured every 15 minutes, and the filtrate was collected from the hollow fiber membrane module. The concentration of protein contained in the filtrate was measured. Measurement of the protein concentration of the plasma was carried out with the use of an in vitro diagnostic kit (MicroTP-Test Wako, manufactured by Wako Pure Chemical Industries). The average protein leak amount was determined based on the data taken over a lapse of 2 hours, and the protein leak amount (TPL) in terms of 3-L fluid removal was calculated, which was subjected to evaluation. TPL decrement in blood is desirably low, so TPL leak into the dialysate is preferably low (that is, TPL concentration in the dialysate is preferably low).

[Table 2]

| | Aperture ratio of outer surface (%) | Filtration stability ΔTMP (mmHg) | Performance in aqueous system | | | | TPL (g/ 3L) |
|---|---|---|---|---|---|---|---|
| | | | UFR (mL/hr/m²/ mmHg) | CL (mL/min/m²) [MBE (%) | | | |
| | | | | CLun | CLvb | CLmyo | |
| Ex.1 | 31.7 | 9 | 117 | 164 [-1.2] | 88 [-1.9] | 41 [15] | 0.0 |
| Comp.Ex. 1 (conventional product) | 0.0 | 17 | 41 | 162 [0.3] | 68 [-9.0] | 22 [15] | 0.1 |
| Comp.Ex.2 | - | - | 271 | 166 [3.6] | 109 [18] | 113 [100] | 0.0 |
| Comp.Ex.3 | - | - | 310 | 148 [0.5] | 81 [-2.6] | 73 [67] | 0.0 |

Results shown in Table 2 indicate that ΔTMP is lower in Example 1 than in Comparative Example 1. Also, results shown in Fig. 6 indicate that over-time increase of ΔTMP is smaller in Example 1 than in Comparative Example 1. These results indicate that Example 1 exhibits excellent filtration stability.

Preferably, the ΔTMP value measured after a lapse of 150 minutes is 15 mmHg or less (that is, equal to or less than the value obtainable with a conventional PES membrane).

It is also conceivable that Example 1 exhibits a higher UFR than Comparative Example 1 and has properties suitable for use in CRRT and the like.

Preferably, MBE of all the above-mentioned clearance (CL) is low (see The method of hemodialyzer performance assessment, 2012, Journal of Japanese Society for Dialysis Therapy 45(5):435-445). For example, preferably, MBE of CLun (urea clearance) is within the range of ±5%. Preferably, MBE of CLvb (vitamin B12 clearance) is within the range of ±10%. Preferably, MBE of CLmyo (myoglobin clearance) is within the range of ±30%. Example 1, like Comparative Example 1, exhibits all the clearances (CLun, CLvb, and CLmyo) within the preferable ranges described above and is conceivable to have properties suitable for use in CRRT and the like.

Fig. 7 shows the relationship between the PVP content and the CLmyo MBE in Example 1 and Comparative Examples 1, 2. Referring to Fig. 7, the higher the PVP content is, the lower the CLmyo MBE is.

Example 1 exhibits a lower TPL leak amount than Comparative Example and is conceivable to have properties suitable for use in CRRT and the like.

## Claims

1. A semipermeable membrane comprising:

   a polyether sulfone;
   a sulfonated polyarylene ether copolymer; and
   polyvinylpyrrolidone, wherein
   the sulfonated polyarylene ether copolymer includes a hydrophobic segment repeating unit represented by the following formula (1) and a hydrophilic segment repeating unit represented by the following formula (2) as copolymerization components,

[Chemical 1]

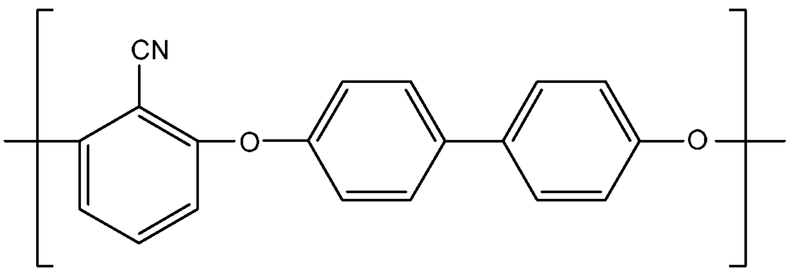

(1)

[Chemical 2]

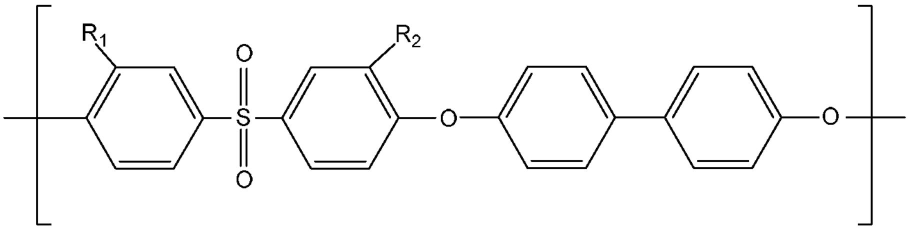

(2)

a component ratio (in mole) of the formula (1) is from 0.50 to 0.65, a component ratio (in mole) of the formula (2) is from 0.35 to 0.50, a sum of the component ratio (in mole) of the formula (1) and the component ratio (in mole) of the formula (2) is 1.00, each of $R_1$ and $R_2$ represents "$-SO_3M$", M represents a metallic element,
a ratio of (the polyether sulfone)/(the sulfonated polyarylene ether copolymer)/(the polyvinylpyrrolidone) (in mass) in the entirety of the semipermeable membrane is (75 to 90)/(7 to 22)/(3 to 18), and
the semipermeable membrane has cytokine adsorption capabilities suitable for continuous renal replacement therapy.

**2.** The semipermeable membrane according to claim 1, wherein the semipermeable membrane has a structure that is not uniform in a thickness direction.

**3.** The semipermeable membrane according to claim 1 or 2, wherein the semipermeable membrane has a dense layer on an inner surface side thereof.

**4.** The semipermeable membrane according to any one of claims 1 to 3, wherein a NaOH titration amount for the entirety of the semipermeable membrane is from 1.2 to 3.0 mL.

FIG.1

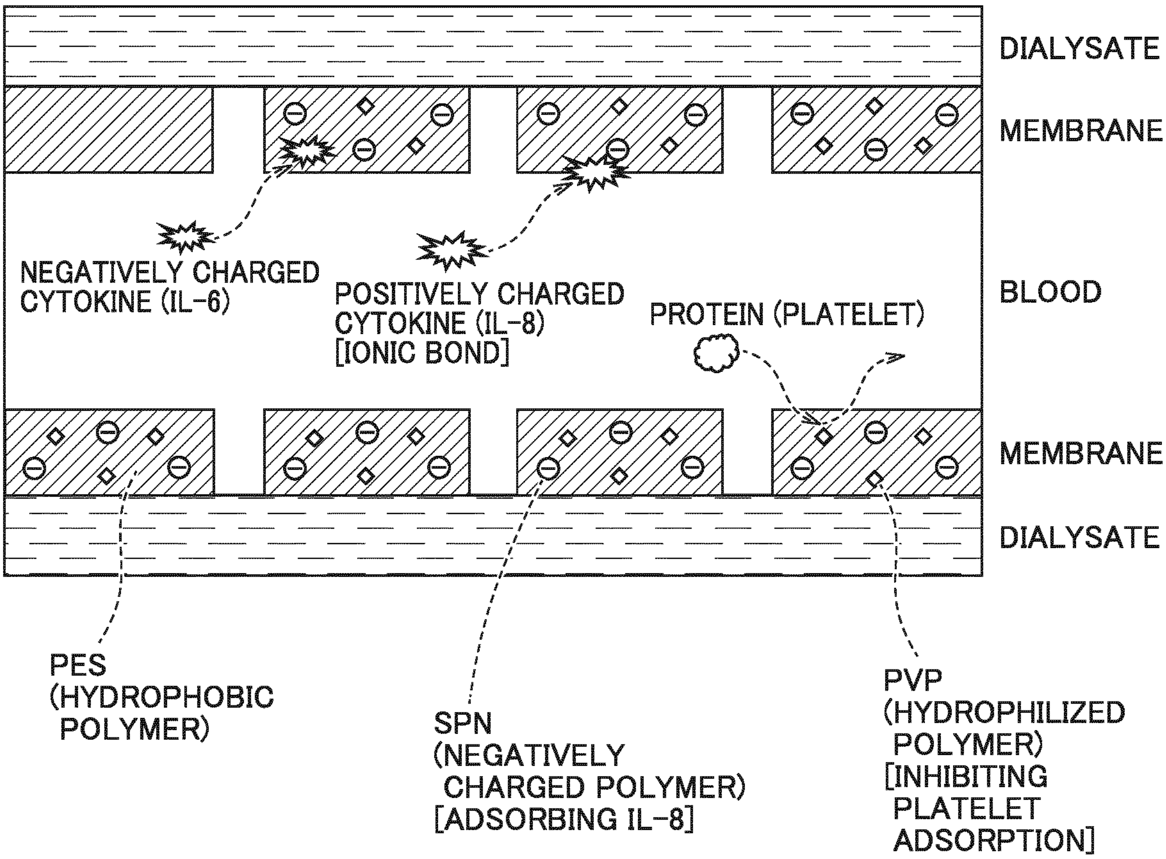
DIALYSATE
MEMBRANE
NEGATIVELY CHARGED CYTOKINE (IL-6)
POSITIVELY CHARGED CYTOKINE (IL-8) [IONIC BOND]
PROTEIN (PLATELET)
BLOOD
MEMBRANE
DIALYSATE
PES (HYDROPHOBIC POLYMER)
SPN (NEGATIVELY CHARGED POLYMER) [ADSORBING IL-8]
PVP (HYDROPHILIZED POLYMER) [INHIBITING PLATELET ADSORPTION]

FIG.2

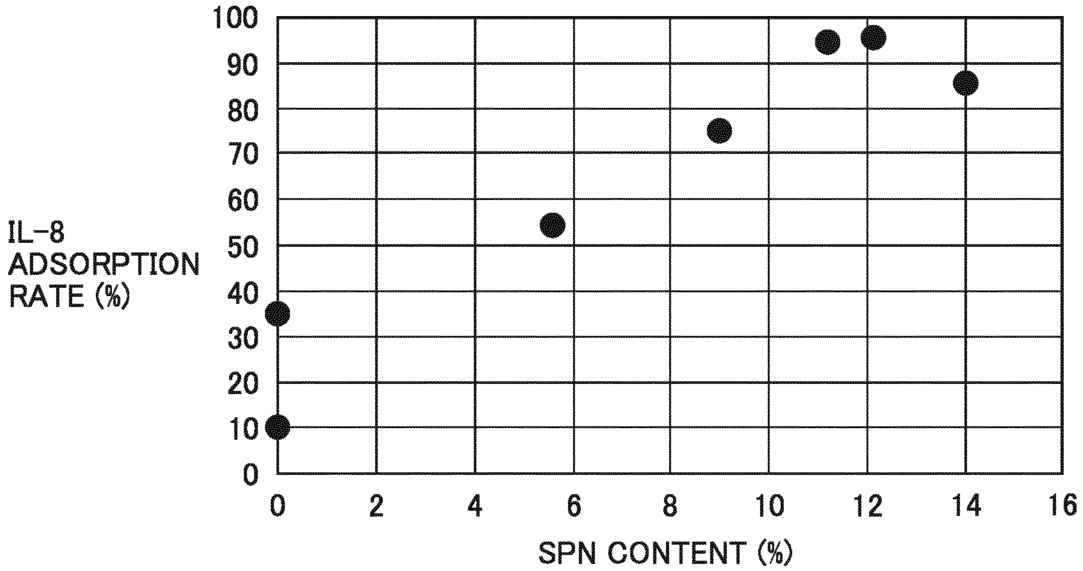
IL-8
ADSORPTION
RATE (%)
100
90
80
70
60
50
40
30
20
10
0
0
2
4
6
8
10
12
14
16
SPN CONTENT (%)

FIG.3

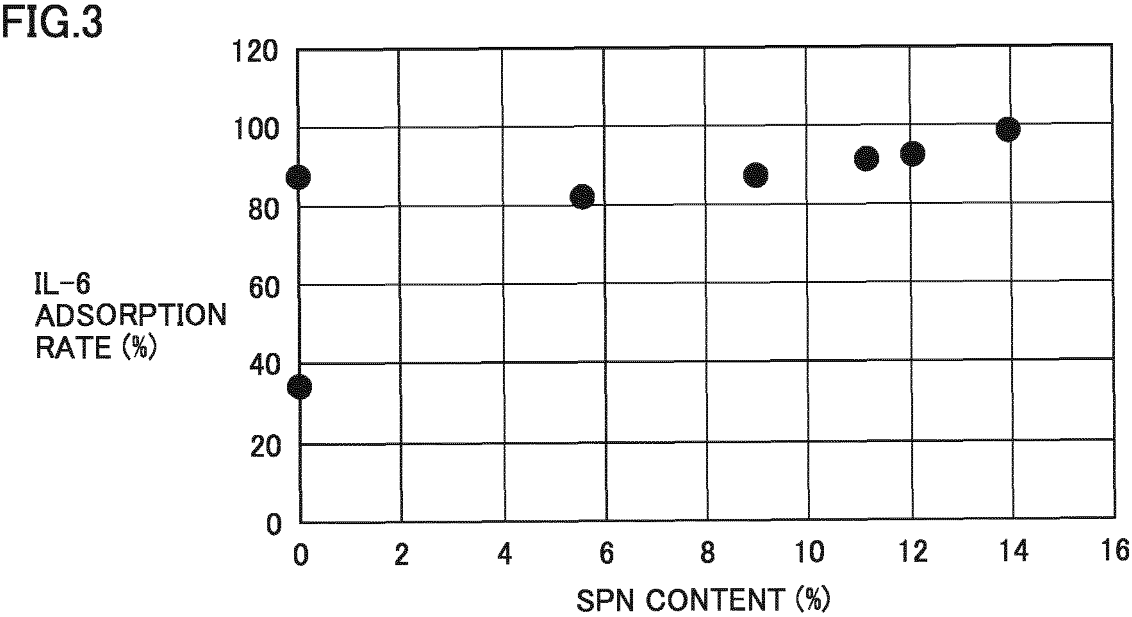
120
100
80
60
40
20
0
IL-6
ADSORPTION
RATE (%)
0
2
4
6
8
10
12
14
16
SPN CONTENT (%)

FIG.4

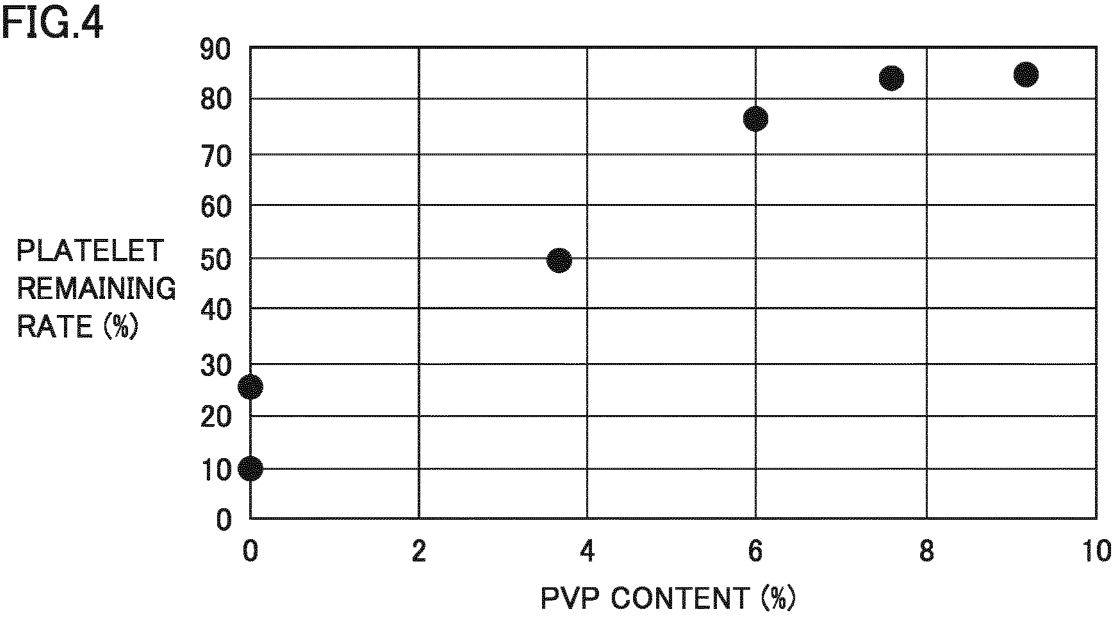
PLATELET
REMAINING
RATE (%)
0
10
20
30
40
50
60
70
80
90
0
2
4
6
8
10
PVP CONTENT (%)

FIG.5

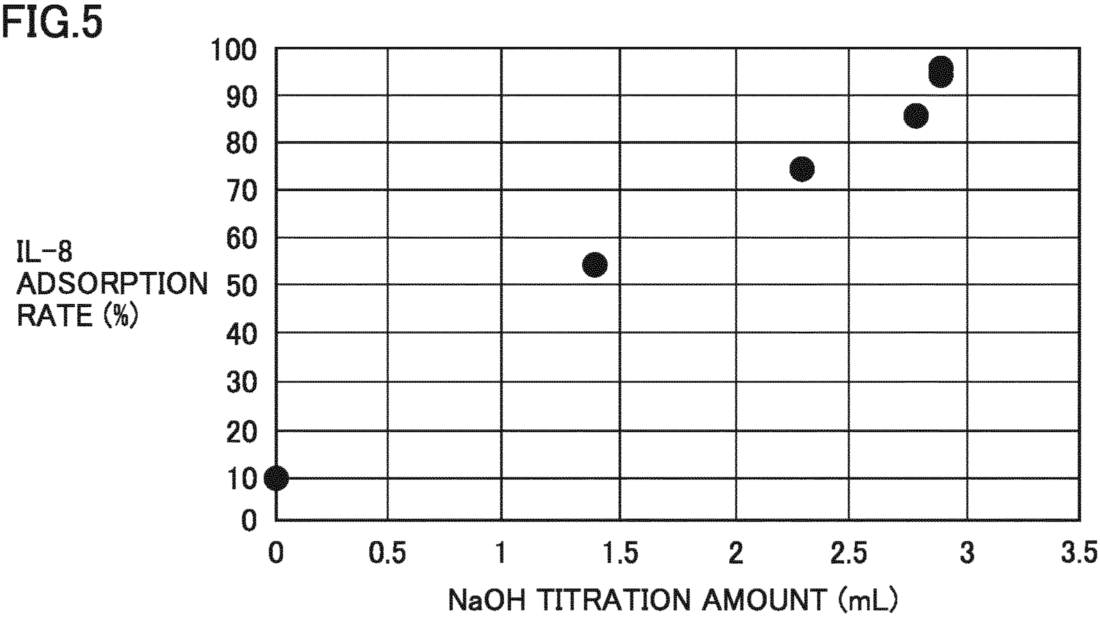
IL-8
ADSORPTION
RATE (%)
0
10
20
30
40
50
60
70
80
90
100
0
0.5
1
1.5
2
2.5
3
3.5
NaOH TITRATION AMOUNT (mL)

FIG.6

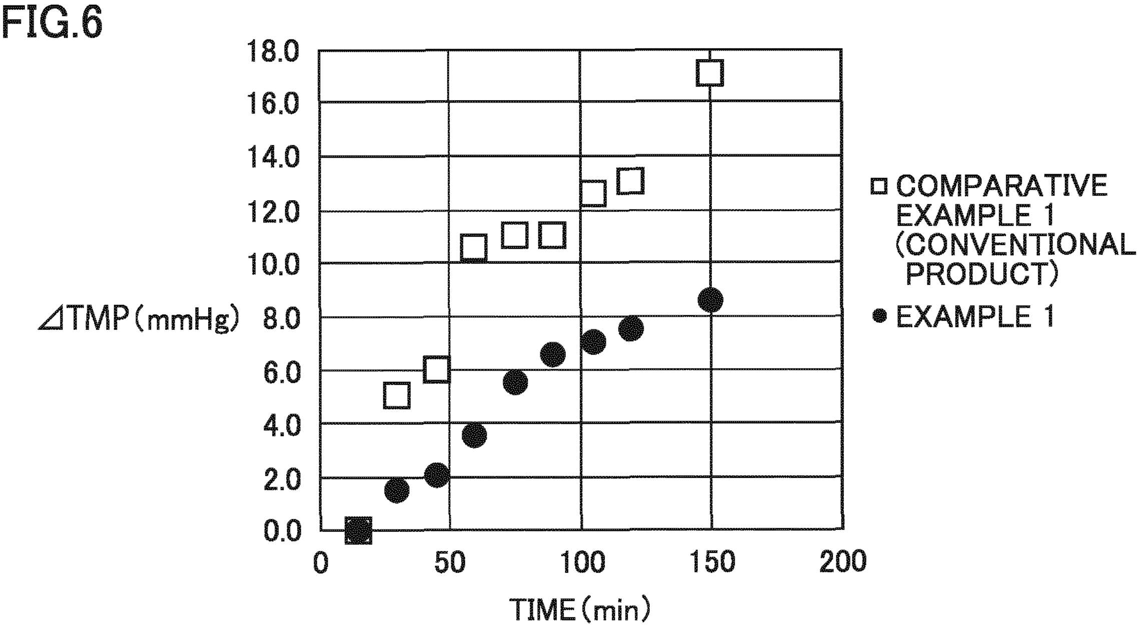
18.0
16.0
14.0
12.0
10.0
⊿TMP (mmHg)
8.0
6.0
4.0
2.0
0.0
0
50
100
150
200
TIME (min)
COMPARATIVE EXAMPLE 1 (CONVENTIONAL PRODUCT)
EXAMPLE 1

FIG.7

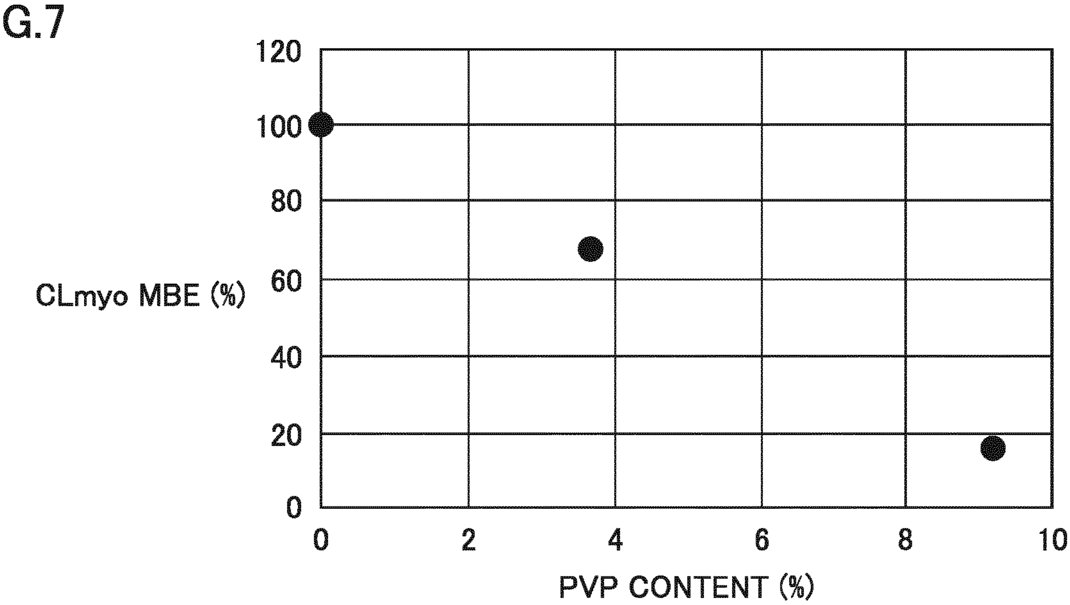

FIG.8

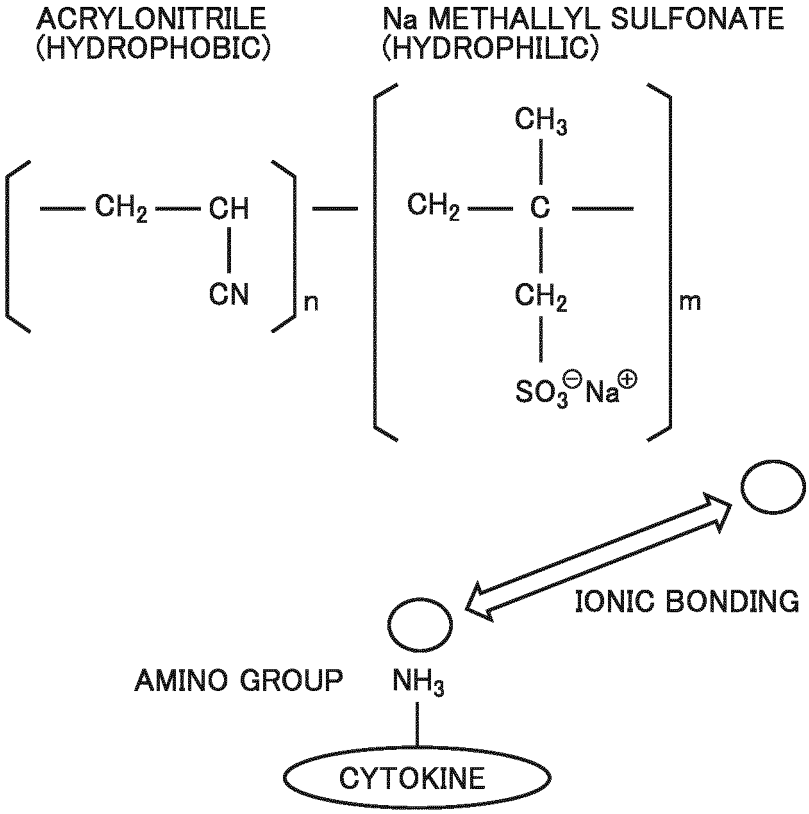
ACRYLONITRILE
(HYDROPHOBIC)
Na METHALLYL SULFONATE
(HYDROPHILIC)
IONIC BONDING
AMINO GROUP
CYTOKINE

FIG.9

| | EXAMPLE 1 |
|---|---|
| OUTER SURFACE | 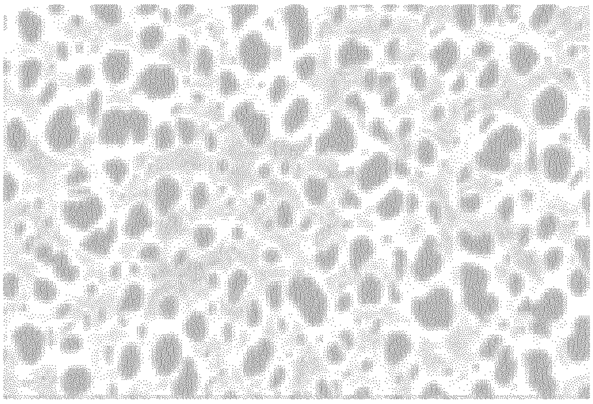 |
| INNER SURFACE | 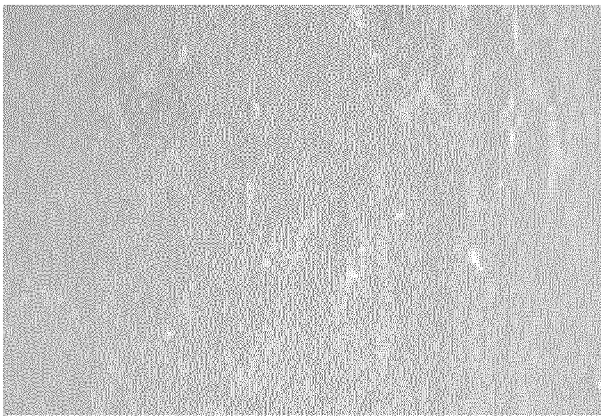 |
| CROSS SECTION | 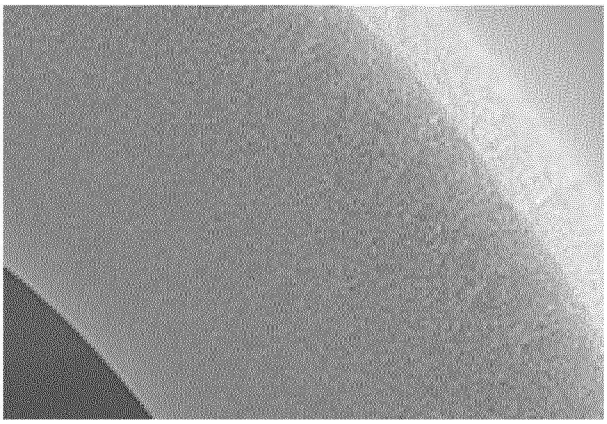 |

# INTERNATIONAL SEARCH REPORT

International application No.
**PCT/JP2022/027231**

## A. CLASSIFICATION OF SUBJECT MATTER

***A61M 1/18***(2006.01)i; ***A61K 31/765***(2006.01)i; ***A61M 1/36***(2006.01)i; ***A61P 7/08***(2006.01)i; ***B01D 71/52***(2006.01)i; ***B01D 71/62***(2006.01)i; ***B01D 71/68***(2006.01)i
FI: A61M1/18 500; A61M1/18 527; A61M1/36 165; B01D71/52; B01D71/62; B01D71/68; A61K31/765; A61P7/08

According to International Patent Classification (IPC) or to both national classification and IPC

## B. FIELDS SEARCHED

Minimum documentation searched (classification system followed by classification symbols)

A61M1/18; A61K31/765; A61M1/36; A61P7/08; B01D71/52; B01D71/62; B01D71/68

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| A | JP 2019-018193 A (TOYOBO CO., LTD.) 07 February 2019 (2019-02-07)<br>entire text, all drawings | 1-4 |
| A | JP 2009-125265 A (KANEKA CORP.) 11 June 2009 (2009-06-11)<br>entire text, all drawings | 1-4 |
| A | WO 2013/022012 A1 (TORAY INDUSTRIES, INC.) 14 February 2013 (2013-02-14)<br>entire text, all drawings | 1-4 |
| A | WO 2020/203927 A1 (ASAHI KASEI MEDICAL CO., LTD.) 08 October 2020 (2020-10-08)<br>entire text, all drawings | 1-4 |
| A | WO 2017/064936 A1 (TOYOBO CO., LTD.) 20 April 2017 (2017-04-20)<br>entire text, all drawings | 1-4 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 August 2022** | **30 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/027231**

| Patent document cited in search report | Publication date (day/month/year) | Patent family member(s) | Publication date (day/month/year) |
|---|---|---|---|
| JP 2019-018193 A | 07 February 2019 | (Family: none) | |
| JP 2009-125265 A | 11 June 2009 | (Family: none) | |
| WO 2013/022012 A1 | 14 February 2013 | US 2014/0206818 A1<br>EP 2742997 A1 | |
| WO 2020/203927 A1 | 08 October 2020 | DE 202020101647 U1<br>CN 113613776 A | |
| WO 2017/064936 A1 | 20 April 2017 | US 2019/0076791 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011062282 A
- JP 2001070767 A


### Non-patent literature cited in the description


- *The Journal of Artificial Organs,* 2014, vol. 43 (3), 233-237
- *J. Chromatogr. A,* 2019, vol. 1559, 152-160
- *Journal of Japanese Society for Dialysis Therapy,* vol. 45 (5), 435-445